# EUROPÄISCHE PATENTSCHRIFT

(11) **EP 0 538 657 B1**
(45) Veröffentlichungstag und Bekanntmachung des Hinweises auf die Patenterteilung: **06.03.1996**
(21) Anmeldenummer: 92116799.5
(22) Anmeldetag: 01.10.1992
(51) Int. Cl.: A61F 13/15

(54) **Windelhose**
Diaper
Couche-culotte

(30) Priorität: 02.10.1991 DE 9112276 U
(43) Veröffentlichungstag der Anmeldung: 28.04.1993
(73) Patentinhaber: Ziegler, Lothar, D-93354 Biburg (DE)
(72) Erfinder: Ziegler, Lothar, D-93354 Biburg (DE)
(74) Vertreter: Graf, Helmut, Dipl.-Ing.

(56) Entgegenhaltungen:
- EP-A- 0 446 867
- DE-U- 9 105 907
- US-A- 3 882 871
- US-A- 4 496 360
- US-A- 4 801 298

## Beschreibung

Die Erfindung bezieht sich auf eine Windelhose gemäß Oberbegriff Patentanspruch 1 oder 2.

Zumindest in den Industrieländern ist die Verwendung von Einmal- bzw. Wegwerf-Windeln weit verbreitet. Die Herstellung, aber auch die Entsorgung dieser Windeln stellt zunehmend ein Problem dar, insbesondere auch wegen der sehr unterschiedlichen, bei Einmal-Windeln verwendeten Materialien bzw. Kunststoffe (siehe EP-A-0 446 867).

Der Erfindung liegt die Aufgabe zugrunde, eine brauchbare Alternative zu den bisherigen Einmal-Windeln aufzuzeigen.

Zur Lösung dieser Aufgabe wird eine Windel vorgeschlagen, die entsprechend dem kennzeichnenden Teil des Patentanspruches 1 ausgeführt ist.

"Mikrofaser-Material" ist im Sinne der Erfindung ein atmungsaktives und dennoch eine Sperre gegen Flüssigkeiten bildendes Material, wie es beispielsweise unter der Markenbezeichnung "GORE-TEX" auf dem Markt angeboten wird.

Die erfindungsgemäße Windelhose garantiert hohe Sicherheit, läßt sich mit den üblichen Waschverfahren hygienisch reinigen und kann daher mehrfach verwendet werden. Hierdurch ergibt sich u.a. nicht nur eine erhebliche Reduzierung der Verbraucherkosten, sondern vor allem auch eine Reduzierung der Umweltbelastung und des Müllanfalls.

Weiterbildungen der Erfindung sind Gegenstand der Unteransprüche.

Die Erfindung wird im folgenden anhand der Figuren an Ausführungsbeispielen näher erläutert. Es zeigen:
- Fig. 1: in vereinfachter Darstellung eine Windelhose eines Windelhosensets gemäß der Erfindung, zusammen mit einer Windeleinlage;
- Fig. 2: in gleicher Darstellung wie Fig. 1 die zugehörige Traghose des Windelhosensets gemäß der Erfindung;
- Fig. 3: einen Schnitt entsprechend der Linie I-I der Fig. 2;
- Fig. 4: in ähnlicher Darstellung wie Fig. 2 eine Windelhose, zusammen mit einer Windeleinlage bei einer weiteren Ausführungsform der Erfindung;
- Fig. 5: in Teildarstellung und in Seitenansicht die teilweise geschlossene Windelhose nach Fig. 4.

In den Figuren ist 1 eine Windelhose, die im wesentlichen aus zwei Lagen eines Zuschnitts aus einem Baumwollgewebe gebildet ist, welche (Lagen) am Umfang der Windelhose 1 sowie auch an weiteren Bereichen durch Versteppen miteinander verbunden sind.

Die Windelhose 1 bildet ein Rückenteil 2 mit zwei Lappen 3 zum Schließen und Festlegen der Windelhose. Weiterhin bildet die Windelhose 1 ein Vorderteil 4, der zwei Lappen 5 aufweist und beim Anlegen der Windelhose auf den Bauch des zu wickelnden Kindes nach oben geklappt wird und an dem ebenso wie an den beiden Lappen 3 die passenden Elemente 6 eines Klettverschlusses durch Aufnähen befestigt sind. Im Beinchenbereich 7, aber auch oben am Rückenteil 2 sowie unten in der Mitte des Vorderteils 4 ist jeweils ein Gummizug 8, 9 bzw. 10 eingearbeitet. Im mittleren Teil ist die Windelhose 1 zwischen den beiden von dem Baumwollgewebe gebildeten Lagen mit einer saugfähigen Zwischenschicht versehen, die bei der dargestellten Ausführungsform ein eingenähtes Vlies aus einer saugfähigen Naturfaser, beispielsweise aus Baumwolle und aus Zellulose ist.

In die Windelhose 1 kann eine Einlage 11 eingelegt werden, die ebenfalls aus zwei Zuschnitten aus einem Baumwollgewebe besteht. Zwischen diesen an ihrem Umfang wiederum miteinander verbundenen Zuschnitten weist die Einlage 11 ein eingenähtes Innenvlies aus einem saugfähigen Naturmaterial, beispielsweise aus Zellulosefasern auf.

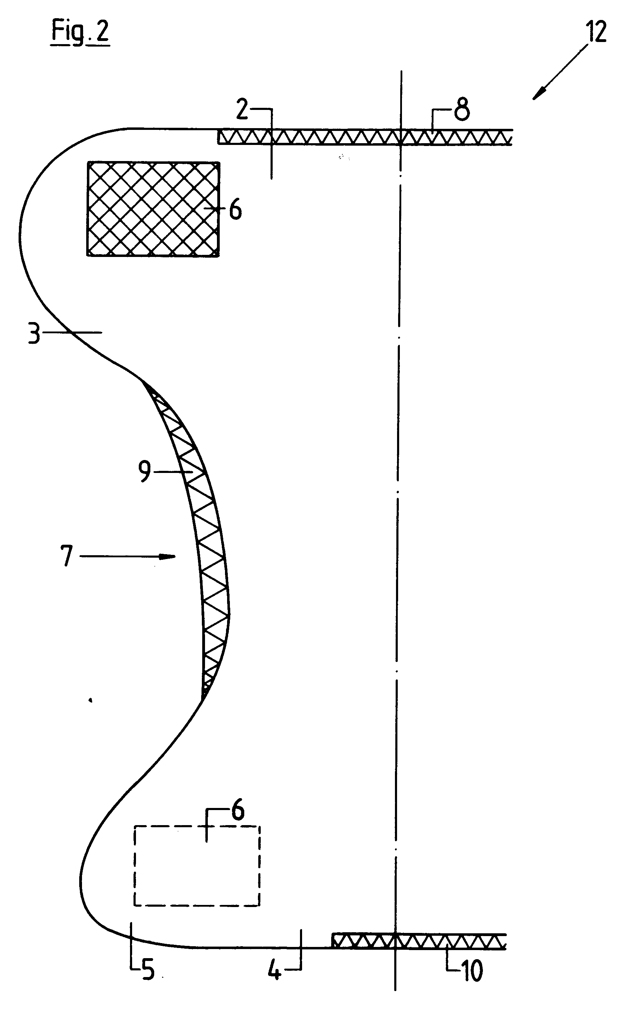

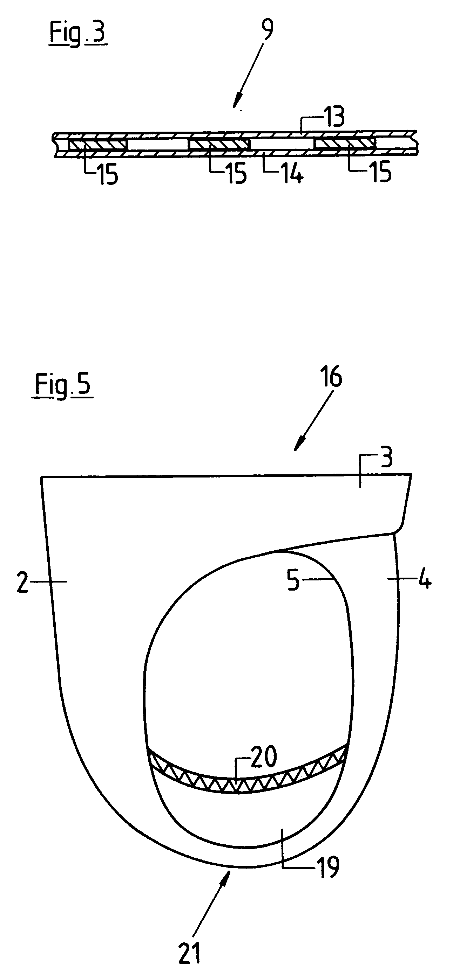

Neben der Windelhose 1 und der Einlage 11 besteht das Windelhosenset weiterhin aus der in den Figuren 2 und 3 mehr im Detail dargestellten Traghose 12, die im wesentlichen gleich der Windelhose 1 geformt ist, allerdings aus einer Mikrofaser hergestellt ist, d.h. aus einem Material, welches aus Nässesperre wirkt, d.h. einen Nässedurchtritt nach außen verhindert, dennoch aber atmungsaktiv ist. Materialien dieser Art werden beispielsweise unter der Bezeichnung "GORE-TEX" auf dem Markt angeboten. Die Traghose 12 besitzt wiederum den Rückenteil 2 mit den beiden Lappen 3, den Vorderteil 4 mit den beiden Lappen 5 sowie die Elemente 6 der Klettverschlüsse an den Lappen 3 und 5. Weiterhin sind auch die Gummizüge 8 und 10 oder entsprechende Gummizüge vorgesehen. Eine Besonderheit besteht darin, daß die Traghose 12 im Beinchenbereich 7 mehrlagig ausgebildet ist. Der dortige Gummizug 9 besteht aus mehreren zwischen den beiden Lagen 15 vorgesehenen Gummibändern 15, von denen bei der dargestellten Ausführungsform drei oder mehr als drei Gummibänder 15 vorgesehen sind, so daß sich nach dem Anlegen der Traghose 12 trotz hoher Bewegungsfreiheit sich ein besonders dichter, auch an die Körperform optimal anpassender Abschluß im Beinbereich ergibt.

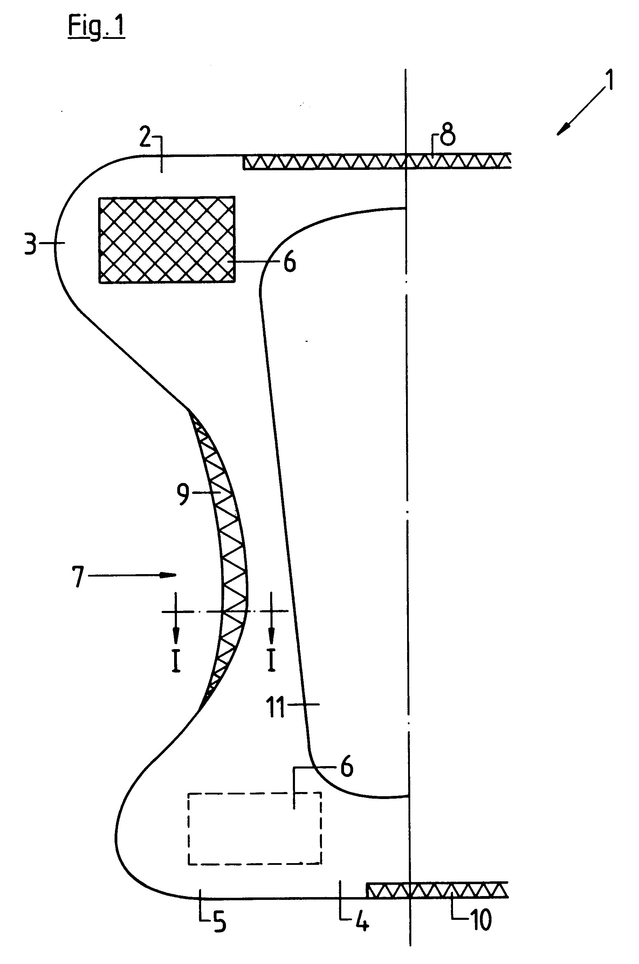

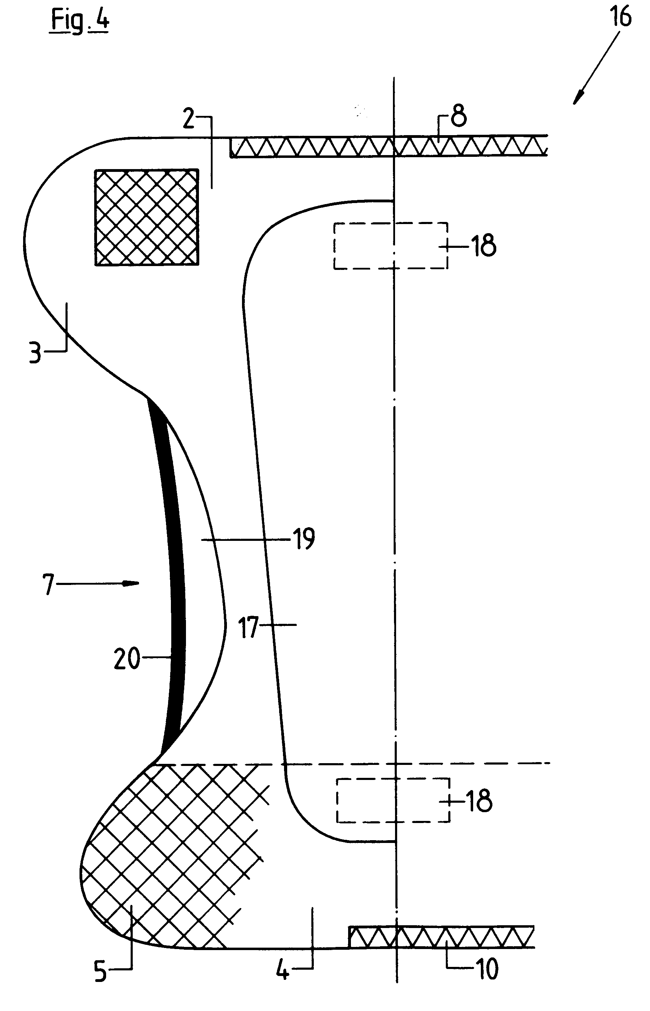

Während das aus der Windelhose 1, der Einlage 11 und der Traghose 12 bestehende Windelhosenset insbesondere für den Heimsektor bestimmt ist, zeigen die Figuren 4 und 5 eine Windelhose 16, die insbesondere auch für den Klinikbereich geeignet ist. Diese Windelhose 16 entspricht in ihrem Zuschnitt der Windelhose 1, besteht aber wie die Traghose 12 aus dem Mikrofaser-Material. Die der Windelhose 1 bzw. der Traghose 12 entsprechenden Elemente der Windelhose 16 sind wiederum mit den gleichen Bezugsziffern wie in den Figuren 1 und 2 bezeichnet. Eine Besonderheit der Windelhose 16 besteht darin, daß die mit dieser Windelhose verwendete Einlage 17 mit Klettverschlüssen in der Windelhose 16 festlegbar ist. Eine weitere Besonderheit besteht auch darin, daß im Beinchenbereich 7 an beiden Seiten jeweils ein Einsatz 19 eingenäht ist, der ebenfalls aus einem Zuschnitt aus dem Mikrofaser-Material besteht. An dem außen liegenden Rand jedes Einsatzes 19 ist dann ein Gummizug 20 vorgesehen.

Wie in der Fig. 5 dargestellt ist, ergeben sich nach dem Anlegen der Windelhose 16 durch die beiden Einsätze 19 im Beinbereich seitliche Faltentaschen 21, die eine extrem große Bewegungsmöglichkeit schaffen und trotzdem einen dichten Abschluß gewährleisten. Durch diese Ausbildung ist es möglich, die Windelhose 16 mit einer bestimmten, vorgegebenen Größe für unterschiedlich große bzw. unterschiedlich alte Babys zu verwenden, d.h. durch die beschriebene Ausbildung kann die benötigte Anzahl unterschiedlicher Größen der Windelhose 16 erheblich reduziert werden, so daß beispielsweise für den gesamten Klinikbereich allenfalls zwei oder drei unterschiedliche Größen für die Windelhose 16 notwendig sind.

Bei der Windelhose 16 ist das am Vorderteil 4 vorgesehene Element 6 des Klettverschlusses extrem breit ausgebildet, d.h. dieses Element erstreckt sich praktisch über die gesamte außen liegende Fläche des Vorderteiles 4, was ebenfalls entscheidend dazu beiträgt, daß die Windelhose 16 einer bestimmten Größe für Babys unterschiedlichen Alters bzw. Größe verwendet werden kann.

Die Erfindung wurde voranstehend an zwei Ausführungsbeispielen beschrieben. Es versteht sich, daß Änderungen sowie Abwandlungen möglich sind, ohne daß dadurch der der Erfindung zugrundeliegende Erfindungsgedanke verlassen wird. So ist die erfindungsgemäße Windelhose bzw. das erfindungsgemäße Windelhosenset mit entsprechender Größe auch als Inkontinenz-Unterwäsche verwendbar.

Der Vorteil besteht grundsätzlich darin, daß durch die verwendeten Materialien eine zuverlässige und hygienische Reinigung mit den üblichen Waschverfahren, insbesondere auch durch Waschen bei 95 °C möglich ist, die Windelhose bzw. das Windelhosenset zusammen mit den verwendeten Einlagen also mehrfach verwendet werden können, was einen ganz entscheidenden Beitrag zur Reduzierung des Müllaufkommens darstellt. Darüber hinaus trägt die erfindungsgemäße Windel bzw. das erfindungsgemäße Windelhosenset auch entscheidend dazu bei, den bei heute üblicherweise verwendeten Wegwerfwindeln schon bei deren Herstellung benötigten Rohstoffbedarf bzw. die bei der Herstellung unvermeidliche Umweltbelastung erheblich zu reduzieren.

## Patentansprüche

1. Windelhose, hergestellt aus wenigstens einem Zuschnitt eines Flachmaterials, der einen Rückenteil (2) sowie einen Vorderteil (4) und dazwischen liegend einen Beinbereich (7) bildet, wobei zumindest im Beinbereich (7) beidseitig elastische Elemente eingearbeitet sind, dadurch gekennzeichnet, daß bei Ausbildung der Windelhose als wiederverwendbare Windelhose das Flachmaterial ein kochfestes Material, nämlich ein Mikrofaser-Material ist und die elastischen Elemente jeweils ein Gummizug (8) sind, der von wenigstens drei voneinander beabstandeten und parallel zueinander angeordneten Gummibändern (15) gebildet ist, die zwischen zwei Lagen (13, 14) des Flachmaterials vorgesehen sind.

2. Windelhose nach Anspruch 1, dadurch gekennzeichnet, daß die Windelhose (16) am Beinbereich (7) jeweils an beiden Seiten einen Einsatz (19) aufweist, der an seinem freiliegenden Rand mit dem Gummizug (20) versehen ist und bei angelegter Windelhose (16) eine Faltentasche (21) bildet.

3. Windelhose nach Anspruch 1 oder 2, dadurch gekennzeichnet, daß die Windelhose eine Traghose (12) einer Windelhosenkombination ist, welches zusätzlich zu der aus dem Mikrofasermaterial hergestellten Traghose (12) eine vorzugsweise mehrlagige Windelhose (1) aus einer Naturfaser, bevorzugt aus Baumwolle, sowie eine Einlage (11) aus Naturfaser, bevorzugt aus Baumwolle aufweist.

4. Windelhose nach einem der Ansprüche 1 bis 3, dadurch gekennzeichnet, daß die Windelhose (16) Klettverschlüsse (18) zum Befestigen einer Einlage (17), vorzugsweise einer mehrlagigen, aus Naturfaser hergestellten Einlage aufweist.

## Claims

1. Diaper pant produced out of at least one cut of a flat material which forms a back part (2) a front part (4) and in between, a crotch (7), whereby at least in the crotch (7) elastic parts are worked in at both sides and it is characteristic that when designing the diaper pant as a reusable diaper pant the flat material used is a micro fibre, which can be boiled, and the elastic parts consist of an elastic band (8) which is formed out of at least two elastic bands (15) laid parallel to one another with a space in between them, that are placed between the two layers (13, 14) of the flat material.

2. Diaper pant according to claim 1, characterised by the diaper pant (16) having an elastic band on the front (10) and at the back (8) which hold the slip insert in place when the diaper pant is applied.

3. Diaper pant according to claim 1 or 2, characterised by the diaper pant being a pant (12), which is a combination diaper pant consisting of a multi layer diaper pant out of a natural fibre, such as cotton or rayon, including a slip insert also made of a natural fibre such as cotton, and covered by a micro fibre pant which prevents moisture from escaping but allows air to circulate and regulates the temperature.

4. Diaper pant according to one of the claims 1 to 3, characterised by the diaper pant (16) having Velcro bands (18) for keeping the insert in place (17) preferably a multi layer insert made out of natural fibres.

## Revendications

1. Couche-culotte réalisée à partir d'au moins une coupe d'un matériau plat formant une partie dorsale (2) ainsi qu'une partie avant (4) et entre elles, un entrejambes (7), et comportant au moins au niveau de l'entrejambes (7) des éléments élastiques insérés des deux côtés, caractérisés par le fait que, lors de la formation de la couche-culotte en tant que couche réutilisable, le matériau plat est fait d'un matériau supportant l'ébullition, à savoir d'un matériau en micro-fibres et les éléments élastiques composés d'une bande en caoutchouc (8) qui est constituée elle-même d'au moins de deux ou trois rubans en caoutchouc (15) séparés et placés de facon parallèle, et prévus entre deux couches (13, 14) du matériau plat.

2. Couche-culotte selon demande 1, caractérisée par le fait que la couche-culotte (16) munite d'un ruban en caoutchouc (20) sur sa bordure dégagée comporte dans l'entrejambes un gousset (19), et qu'elle forme une poche plissée (21) quand la couche culotte (16) est portée.

3. Couche-culotte selon demande 1 ou 2, caractérisée par le fait que la couche-culotte est la culotte portable (12) d'une combinaison couche-culotte qui évite gráce à la circulation d'air une irritation de la peau, composée, outre de la culotte portable fabriquée en micro-fibres, d'une couche-culotte (1) composée elle-même de préférence de plusieurs couches d'une fibre naturelle, de préférence du coton ainsi qu d'une pièce intercalaire (11) en fibre naturelle, de préférence du coton.

4. Couche-culotte selon une des demands 1 à 3, caractérisée par le fait que la couche-culotte (16) montre des bande-velcro (18) pour fixer une pièce intercalaire (17), de préférence une pièce intercalaire composée des plusieurs couches en fibres naturelles.
